# FASCICULE DE BREVET EUROPEEN

(11) **EP 3 651 679 B1**
(45) Date de publication et mention de la délivrance du brevet: **08.12.2021**
(21) Numéro de dépôt: 18749034.7
(22) Date de dépôt: 11.07.2018
(51) Int. Cl.: A61B 46/00, A61M 1/00, A61B 46/23, A61F 13/02

(54) **ENSEMBLE COMPORTANT UN DISPOSITIF ASPIRANT ADAPTÉ POUR ÊTRE PLACÉ SUR UNE PLAIE ET/OU UNE INCISION**
ANORDNUNG MIT EINER SAUGVORRICHTUNG, DIE AUF EINE WUNDE UND/ODER EINEN EINSCHNITT GELEGT WERDEN KANN
ASSEMBLY COMPRISING A SUCTION DEVICE SUITABLE FOR BEING PLACED ON A WOUND AND/OR AN INCISION

(30) Priorité: 10.07.2017 FR 1756504
(43) Date de publication de la demande: 20.05.2020
(73) Titulaire: Touati, Gilles, 80000 Amiens (FR)
(72) Inventeur: Touati, Gilles, 80000 Amiens (FR)
(74) Mandataire: Roman, Alexis
(86) Numéro de dépôt international: PCT/FR2018/051755
(87) Numéro de publication internationale: WO 2019/012228

(56) Documents cités:
- WO-A1-2009/080922
- US-A- 3 763 857
- US-A1- 2005 028 828
- US-A1- 2006 065 275
- US-A1- 2014 309 497
- US-A1- 2015 209 492
- US-B1- 7 252 089

## Description

### Domaine technique de l'invention.

L'invention a pour objet un ensemble comportant un dispositif aspirant d'une plaie et/ou d'une incision d'un patient ainsi que son procédé d'installation sur ledit patient, en vue d'assèchement et de récupération des pertes sanguines.

Elle concerne le domaine technique des accessoires médicaux permettant d'assainir et/ou d'assécher une incision et/ou une plaie lors d'une intervention chirurgicale et/ d'une intervention d'urgence. Elle concerne plus particulièrement le domaine technique des champs opératoires.

### État de la technique.

De nombreux champs opératoires existants peuvent être utilisés lors d'interventions. Par exemple, lors d'opérations de chirurgie, il est courant d'employer des champs opératoires équipés de fenêtres d'incision au travers desquelles sont réalisés les actes chirurgicaux. Ces champs se placent sur la peau du patient et ont pour but d'isoler et de protéger la zone d'incision contre toute contamination. Ils forment une barrière efficace entre le corps du patient et l'atmosphère du bloc opératoire.

Ces champs opératoires ne sont pas totalement efficaces dans la mesure où ils ne permettent pas d'assainir et/ou d'assécher efficacement le site opératoire. En effet, dès que la peau est incisée, des sécrétions d'incision apparaissent (saignements et/ou sécrétions corporelles) qui sont susceptibles d'endommager les berges d'incision et causer des infections de paroi et/ou du site opératoire, notamment des infections nosocomiales. Les pertes sanguines observées peuvent être importantes et générer une anémie par spoliation. De plus, ces sécrétions masquent certaines parties du site opératoire et donc entravent le bon déroulement de l'intervention chirurgicale. Il est donc nécessaire d'évacuer les sécrétions d'incision, généralement en utilisant des compresses absorbantes que l'on change régulièrement une fois qu'elles sont totalement imbibées. L'absorption des sécrétions par compresses induit des manipulations supplémentaires autour du site opératoire susceptibles de gêner le praticien dans l'exécution des actes chirurgicaux. Ces compresses absorbantes sont ensuite jetées et aucune récupération de ce volume de sang n'est possible.

La compresse stérile rapportée à l'extrémité du drap chirurgical décrit dans le document brevet US 4.089.331 (KENDALL & CO), ne permet pas d'absorber efficacement les sécrétions d'incision. En effet, dès que la compresse est totalement imbibée, l'absorption des sécrétions ne peut plus être réalisée de sorte qu'il faut continuer à utiliser des compresses absorbantes supplémentaires et donc continuer à effectuer des manipulations supplémentaires autour du site opératoire.

Dans le cas d'une plaie, on connaît par le document brevet WO 2003/018098 (KCI LICENSING INC), un système destiné à accélérer la cicatrisation d'un tissu difficilement cicatrisable. Ce système comprend un tampon poreux introduit dans une plaie ainsi qu'un pansement étanche à l'air fixé sur ce tampon, permettant une fermeture hermétique de la plaie. Une extrémité proximale d'un conduit est raccordée au pansement, une extrémité distale de ce conduit pouvant être raccordée à une source de dépression, telle qu'une pompe électrique. Un collecteur installé sur le conduit permet de retenir les exsudats aspirés à partir de la plaie pendant l'application d'une dépression. Bien qu'efficace, ce système est complexe, et ne protège pas la plaie contre d'éventuelles infections nosocomiales, et n'a pas de vertu préventive.

Le document brevet WO 2009/080922 (TOUATI) propose de pallier à certains inconvénients précités. Il décrit un dispositif aspirant est adapté pour être placé sur une incision et/ou une plaie d'un patient et destiné à assainir un site opératoire. Ce dispositif comporte un champ opératoire composé d'une couche externe comportant une face supérieure et une face inférieure, et d'une couche interne comportant également une face supérieure et une face inférieure. La face inférieure de la couche interne est aménagée de manière à venir au contact de la plaie et/ou de l'incision. Bien que ce dispositif constitue une amélioration par rapport à l'état de l'art, l'aspiration effectuée par ce type de dispositif est extrêmement limitée et ne permet pas en aucun cas d'auto transfuser le sang du patient lors de l'intervention.

Le document US 3,763,857 (Schrading) porte sur un champ chirurgical du type comportant un dispositif aspirant adapté pour être placé sur une incision et/ou une plaie située sur un membre ou sur le corps d'un patient pour assainir et/ou assécher ladite incision ou ladite plaie. Ce document divulgue les caractéristiques du préambule de la revendication 1.

Le document US 2005/028828 A1 (Heaton Keith Patrick et al.) concerne un dispositif de traitement de plaies par pression négative comprenant un tampon de mousse polymère à cellules ouvertes flexible destiné à être placé sur une plaie à traiter par pression négative et un champ chirurgical destiné à être disposé pardessus le tampon de mousse, lequel champ chirurgical est équipé d'une tête d'aspiration comprenant une partie bridée sensiblement plane définissant une surface intérieure qui est destinée à se trouver en regard du tampon de mousse polymère ; un raccord tubulaire ayant une ouverture qui est à même de se raccorder à un tube dans lequel une pression négative peut être produite ; et une pluralité de saillies sur la surface de ladite partie bridée, définissant une pluralité de canaux dirigeant un écoulement de fluides de la surface intérieure vers l'ouverture du raccord tubulaire.

L'invention vise à remédier à cet état des choses. En particulier, un objectif de l'invention est de proposer un dispositif capable d'assainir les berges de la plaie et/ou d'incision plus efficacement que les solutions proposées dans l'art antérieur.

Un autre objectif est de proposer un dispositif pouvant être utilisé en urgences et permettant de réutiliser le sang perdu par le patient, lors d'une plaie traumatique.

Encore un objectif est de proposer un dispositif facile à utiliser et sans gène par le praticien.

### Divulgation de l'invention.

La solution proposée par l'invention est un ensemble selon la revendication 1.

Grâce à ce dispositif, le champ opératoire peut être enroulé rapidement autour d'un membre ou autour du corps d'un patient si les hémorragies sont massives. De par sa conception, la zone aspirante et ses cavités permettent de récolter rapidement un grande volume de sang qui va pouvoir va être recyclé et réutilisé, notamment dans le cas d'une autotransfusion. La plaie et/ou l'incision reste en outre relativement saine et/ou asséchée, de sorte que l'état général d'un patient ou d'un blessé peut être stabilisé.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- la couche interne peut être constituée d'une gaze neutre, de sorte à minimiser les interactions avec les globules rouges,
- la zone aspirante peut être composée d'un lacis de cathéters multi-perforés,
- la zone aspirante peut être composée de mousse multi-perforée,
- la zone aspirante peut être composée d'un réseau de treillis de cathéters,
- le champ opératoire peut être composé d'une partie mâle et d'une partie femelle séparables,
- une fenêtre d'incision totale ou partielle peut être emménagée dans le champ opératoire, de manière à avoir un accès à la plaie et/ou incision.

La présente divulgation concerne aussi un procédé d'installation d'un ensemble selon l'invention. Ce procédé ne fait pas partie de l'invention. Il comprend les étapes consistant à :
- positionner le dispositif de protection aspirant en :
   ∘ plaçant la face inférieure de la couche interne du champ opératoire, face à l'incision et/ou face à la plaie,
   ∘ enroulant le champ opératoire autour du membre ou autour du corps du patient où est située l'incision et/ou la plaie,
- connecter l'organe d'aspiration à l'embout de la tubulure d'évacuation.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une vue schématique en coupe d'un champ opératoire constitutif d'un dispositif aspirant conforme à l'invention,
- la figure 2 est une vue schématique de dessus du champ opératoire de la figure 1, celui-ci étant équipé d'une fenêtre d'incision,
- la figure 3 est une vue schématique de dessus d'un champ opératoire conforme à l'invention, ledit champ opératoire étant constitué d'une partie femelle et d'une partie mâle séparables,
- la figure 4 est une vue schématique d'un patient, le dispositif aspirant de la figure 1 étant installé sous le membre dudit patient,
- la figure 5 est une vue schématique d'un patient, le dispositif aspirant de la figure 1 étant enroulé autour du membre dudit patient,
- la figure 6 est une vue schématique d'un cathéter multiperforé utilisé dans la zone aspirante d'un champ opératoire d'un ensemble selon l'invention,
- la figure 7 est une vue schématique d'un exemple de zone aspirante, celle-ci se présentant sous la forme d'une mousse multiperforée,
- la figure 8 est une vue schématique d'un cathéter de type connu utilisé dans un mode de réalisation de la zone aspirante d'un ensemble selon l'invention.

### Modes préférés de réalisation de l'invention.

L'invention concerne un ensemble comportant un dispositif aspirant d'une plaie et/ou d'une incision destiné à les assainir et/ou les assécher pour récupération sanguine autologue et éventuellement à éviter le développement d'infections. Un tel dispositif aspirant comporte un champ opératoire 1 constitué de deux couches 3, 4 enserrant une zone aspirante 6a, 6b, 6c.

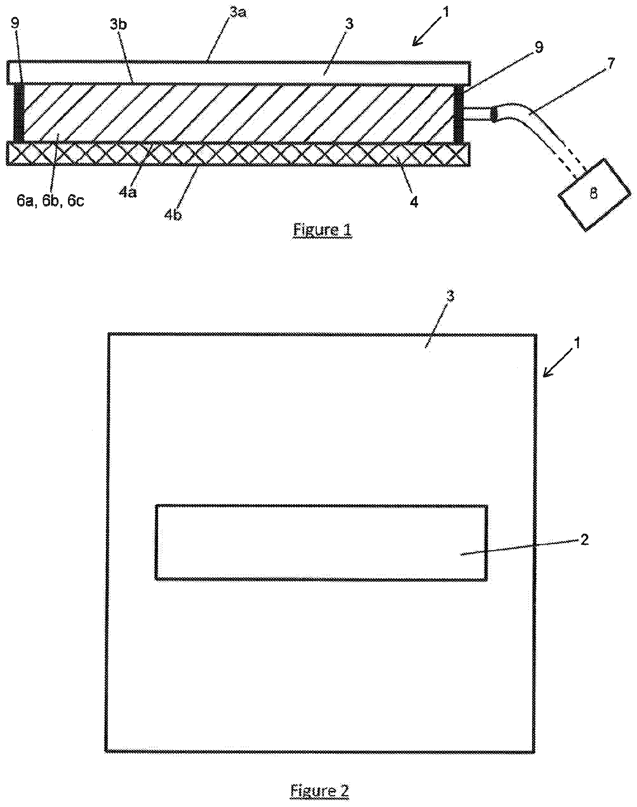

Sur la figure 1, le champ opératoire 1 comporte une zone aspirante 6a, 6b, 6c aménagée entre une couche externe 3 et une couche interne 4.

La couche externe 3 comporte une face supérieure 3a et une face inférieure 3b en contact avec la zone aspirante 6a, 6b, 6c (décrite plus avant dans la description). Cette couche 3 peut, par exemple, être réalisée dans un matériau stérile non tissé du type ouate de cellulose, monocouche ou multicouches. Tout autre matériau connu de l'Homme du métier et convenant à la fabrication d'un champ opératoire 1 peut toutefois être employé.

Préférentiellement et dans le but d'isoler efficacement le corps du patient P de l'environnement extérieur et notamment de l'introduction d'un fluide contaminé, la face supérieure 3a de la couche externe 3 du champ opératoire 1 est préférentiellement imperméabilisée. Cette imperméabilisation peut se faire au moyen d'un film plastique, d'un matériau imperméable aux liquides, d'un matériau hydrophobe, ou encore grâce à tout autre matériau convenant à l'Homme du métier.

La couche interne 4 vient en contact avec le patient P lorsque le dispositif aspirant est installé. Elle permet d'éviter que la zone aspirante 6a, 6b, 6c (décrite plus avant dans la description) ne soit en contact direct avec l'incision et/ou la plaie, et améliore ainsi le confort et la sécurité du patient P. De manière préférentielle, la couche interne 4 se présente sous la forme d'une gaze neutre de manière à ce qu'elle est le moins d'interactions possibles avec les globules rouges contenus dans le sang du patient P. Ainsi, le mode de réalisation préféré pour la couche interne 4 est une gaze neutre de type connu, comme par exemple des compresses stériles.

De la même manière que la couche externe 3, la couche interne 4 peut être réalisée dans un matériau stérile non tissé du type ouate de cellulose, monocouche ou multicouches. Tout autre matériau connu de l'Homme du métier et convenant à la fabrication d'un champ opératoire 1 peut toutefois être employé.

La couche externe 3 ainsi que la couche interne 4 ont chacune une largeur comprise entre 20 cm et 40 cm, une longueur totale comprise entre 20 cm et 60 cm, et une épaisseur variant de 1 mm à 2 mm. Toutefois, ces dimensions ne sont pas limitatives et peuvent être adaptées par l'Homme du métier en fonction du type d'intervention chirurgicale réalisée.

Dans un mode de réalisation préféré, la couche interne 4 et la couche externe 3 sont réalisées en une seule pièce venant enrouler la zone aspirante 6a, 6b, 6c. Une telle configuration permet de faciliter la conception du dispositif de protection aspirant et ainsi limiter les coûts.

La zone aspirante 6a, 6b, 6c est agencée entre la face supérieure 4a de la couche interne 4 et la face inférieure 3b de la couche externe 3. Elle comporte des cavités 61a, 61b, 61c dans lesquelles le sang du patient P va être récolté puis aspiré afin de pouvoir le recycler et ainsi le réutiliser.

La zone aspirante 6a, 6b, 6c est connectée à un organe d'aspiration 8 au moyen d'une tubulure 7. La tubulure 7 est avantageusement perforée sur une partie distale, et se termine, sur sa partie proximale, par un embout destiné à se connecter à l'organe d'aspiration 8. En pratique, on utilise une connectique de type Luerlock^{®} pouvant se connecter à une pompe ou tout autre type d'organe d'aspiration.

L'organe d'aspiration 8 se présente préférentiellement sous la forme d'un système de récupération de sang autologue de type Cell Saver^{®}. Un tel dispositif permet au praticien, après lavage et centrifugation, d'autotransfuser le patient P lors de l'intervention. Une telle caractéristique étant particulièrement utile lors d'interventions en urgence ou encore pour éviter les risques de transfusions allogéniques. Ce système de récupération 8 est préférentiellement portatif, de manière à pouvoir le transporter aisément sur les lieux de l'intervention. Il peut, toutefois, dans le cas d'une intervention ayant lieu dans un bloc opératoire, se présenter sous la forme d'un système fixe mural. Il peut créer une dépression d'aspiration pouvant varier entre -50 mmHg et -300 mmHg, permettant ainsi au praticien de régler la force de l'aspiration en fonction du degré de l'hémorragie.

La zone aspirante 6a, 6b, 6c est étanche, sa périphérie 9 étant fermée de manière à ce que le sang aspiré dans ladite zone aspirante 6a, 6b, 6c ne puisse s'échapper que par la tubulure 7. L'étanchéité peut être réalisée par soudage, ou encore par l'ajout d'un matériau venant enserrer toute la périphérie 9. La zone aspirante 6a, 6b, 6c a des dimensions semblables à celles des couches interne 4 et externe 3. Elle a une longueur comprise entre 20 cm et 60 cm, une largeur variant de 20 cm à 40 cm, et une épaisseur comprise entre 1 mm et 1 cm.

Dans un premier mode de réalisation, la zone aspirante 6a se présente sous la forme d'un lacis de cathéters multiperforés 63a. Ces cathéters 63a sont connus de l'Homme du métier et peuvent être réalisés dans des matériaux tels que le polyamide ou encore le polyuréthane. Le nombre de cathéters multiperforés 63a peut varier suivant la capacité d'absorption nécessaire. La zone aspirante 6a peut, par exemple, comporter entre 1 et 100 cathéters.

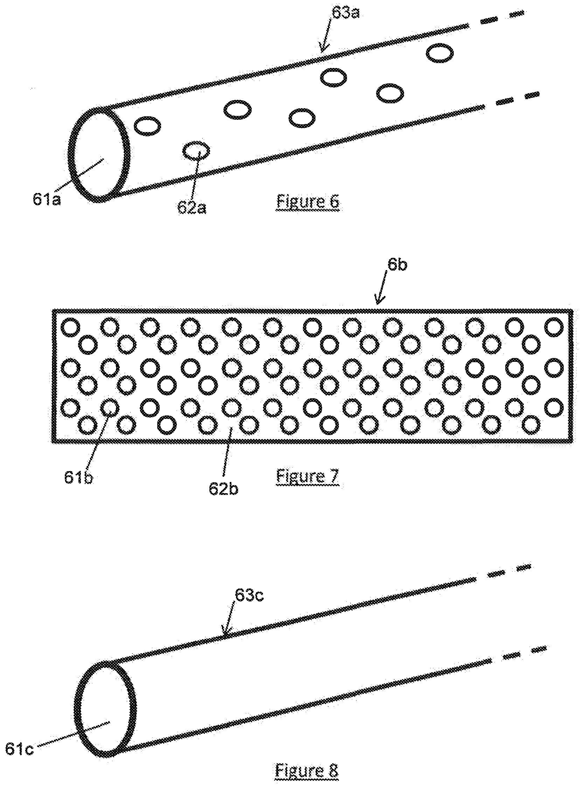

La figure 6 illustre un tel cathéter 63a. Dans ce mode de réalisation, les conduits de chacun des cathéters 63a constituent les cavités 61a dans lesquelles le sang du patient P va être aspiré. La dépression d'aspiration créée par le système 8 va aspirer le sang à travers des perforations 62a de manière à le récupérer dans les conduits 61a et ensuite le transférer audit système de récupération 8.

Un deuxième mode de réalisation représenté sur la figure 7, possède une zone aspirante 6b se présentant sous la forme d'une mousse multiperforée. Cette mousse comporte des cavités 61b, préférentiellement disposées uniformément dans toute la mousse 6b. De la même manière que dans le mode de réalisation précédent, la dépression d'aspiration créée par le système 8 va aspirer le sang dans les cavités 61b à travers la mousse 62b pour ensuite l'envoyer vers ledit système de récupération de sang 8.

Dans un mode de réalisation alternatif, la zone aspirante 6c peut être composée d'un réseau de treillis de cathéters 63c (représenté sur la figure 8). Les treillis peuvent être interconnectés les uns avec les autres ou être séparés. Dans le dernier cas, chacun des treillis sera connecté de façon indépendante au système de récupération 8. De manière similaire à celle décrite dans le premier mode de réalisation, les conduits des cathéters 63c constituent les cavités 61c dans lesquelles le sang aspiré vient se loger. Lorsque la dépression d'aspiration est appliquée grâce au système 8, le sang du patient P est aspiré puis re-transféré audit système de manière à être traité et ensuite re-transfusé.

La figure 2 illustre un mode de réalisation préféré de l'invention dans lequel dispositif aspirant comporte une fenêtre d'incision 2. Celle-ci peut être partielle ou totale, et permet au praticien de réaliser une intervention. Ce mode est surtout utile lorsque le dispositif est utilisé pendant une intervention chirurgicale et qu'il est placé au niveau d'une incision.

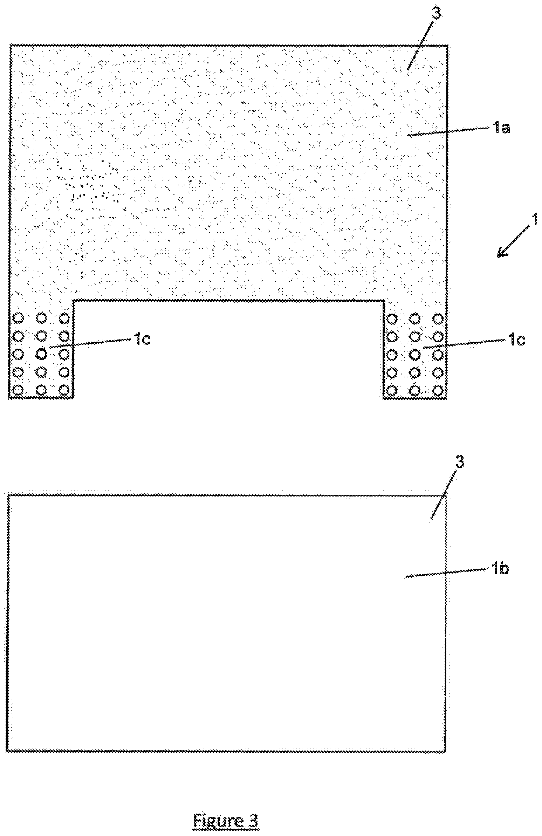

Sur la figure 2, le champ opératoire 1 est réalisé en une seule pièce. Il peut cependant être formé d'une partie mâle 1a et d'une partie femelle 1b séparables (figure 3), la fenêtre d'incision 2 étant aménagée au niveau de la jonction entre ladite partie mâle 1a et ladite partie femelle 1b. L'utilisation d'un champ opératoire 1 en deux parties séparables 1a, 1b simplifie sa conception et sa mise en place par le praticien. De plus, un tel champ opératoire 1 permet de faire varier la taille de la fenêtre d'incision 2 et ainsi permettre au praticien de l'ajuster en fonction de l'opération à effectuer. Pour adapter la taille de la fenêtre d'incision 2 à la taille de l'incision ou de la plaie, la partie mâle 1a et la partie femelle 1b du champ opératoire 1 sont maintenues en position par un dispositif de fixation réglable permettant d'ajuster la position de ladite partie mâle 1a par rapport à ladite partie femelle 1b et de faire varier la taille de ladite fenêtre d'incision 2.

En se référant à la figure 3, le dispositif de fixation réglable est préférentiellement formé de pattes de fixation 1c adhésives agencées sur la partie mâle 1a et aptes à adhérer sur la partie femelle 1b. Les zones adhésives peuvent éventuellement être protégées par un papier de protection amovible que l'opérateur enlèvera juste avant la mise en place. Tout autre dispositif de fixation réglable équivalent peut être utilisé par l'Homme du métier, comme par exemple des bandes à boucles et à crochets de type VELCRO^{®}, ou encore des éléments mâles s'insérant dans des éléments femelles.

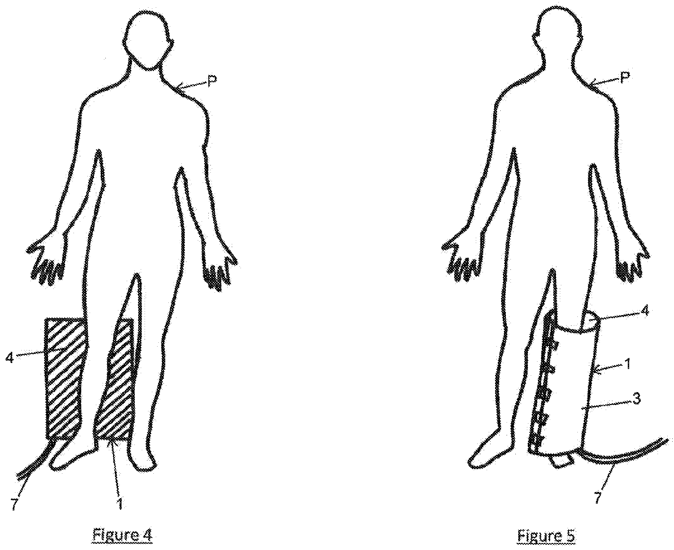

Les figures 4 et 5 représentent des exemples d'installation du dispositif aspirant. Le membre du patient P peut être posé sur le champ opératoire 1 (figure 4). Cette configuration est particulièrement pratique lorsqu'un praticien opère en urgence et/ou lorsque le débit de perte de sang est élevé.

Dans un autre mode de réalisation représenté sur la figure 5, le champ opératoire 1 est enroulé autour du membre ou di corps du patient où est située la plaie et/ou l'incision, permettant ainsi de maximiser la récupération du sang lors d'une intervention. Dans ce mode, le champ opératoire 1 possède préférentiellement un dispositif de fixation 5 permettant de le maintenir en position après l'avoir positionné. Ce dispositif peut, par exemple, se présenter sous la forme de sangles, de Velcro^{®}, ou encore d'un dispositif de type bouton-pressions.

L'enroulement du champ opératoire 1 entraîne l'enroulement simultané de sa couche externe 3 et de sa couche interne 4. Le dispositif aspirant peut donc être mis en place facilement et rapidement, dans des situations d'urgences extrêmes, en une seule étape, contrairement au dispositif décrit dans le document brevet US2005/0028828 (HEATON) dont la mise en place nécessite plusieurs étapes.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de la portée de l'invention qui est définie dans les revendications. En particulier :
- le champ opératoire 1 peut être positionné différemment que les placements décrits précédemment. Il peut, par exemple, être placé au niveau d'un des bras, du torse, etc.,
- la zone aspirante 6a, 6b, 6c peut être différente de celle décrite. Elle peut par exemple se présenter sous la forme d'une combinaison des modes détaillés précédemment,
- la zone aspirante 6a, 6b, 6c peut être équipée de plusieurs tubulures 7, permettant de la connecter simultanément à plusieurs organes d'aspiration 8,
- la forme du champ opératoire 1 peut être différente de celle décrite précédemment. Elle peut varier en fonction de la position de l'incision et/ou de la plaie. Elle peut, par exemple, se présenter sous la forme d'un cercle, d'une ellipse, ou encore toute autre forme convenant à l'Homme du métier.

## Revendications

1. Ensemble comportant :
- un dispositif aspirant adapté pour être placé sur une incision et/ou une plaie située sur un membre ou sur le corps d'un patient (P) pour assainir et/ou assécher ladite incision ou ladite plaie, ledit dispositif comportant un champ opératoire (1) comprenant :
∘ une couche externe (3) comportant une face supérieure (3a) et une face inférieure (3b),
∘ une couche interne (4) comportant une face supérieure (4a) et une face inférieure (4b), laquelle face inférieure (4b) est aménagée de manière à venir au contact de la plaie et/ou de l'incision,
- un organe d'aspiration (8),
- une zone aspirante (6a, 6b, 6c) présentant des cavités (61a, 61b, 61c), laquelle zone aspirante (6a, 6b, 6c) est aménagée entre la face inférieure (3b) de la couche externe (3) et la face supérieure (4b) de la couche interne (4), laquelle zone aspirante (6a, 6b, 6c) a une périphérie (9) étanche,
- une tubulure d'évacuation (7) raccordée à la zone aspirante (6a, 6b, 6c), laquelle tubulure (7) se termine par un embout adapté pour se connecter à l'organe d'aspiration (8) pour créer une dépression d'aspiration dans la zone aspirante (6a, 6b, 6c),
**caractérisé en ce que** :
- la face supérieure (3a) de la couche externe (3) du champ opératoire (1) est imperméable,
- le champ opératoire (1) est adapté pour être enroulé autour du membre ou du corps du patient,
- la couche externe (3) et la couche interne (4) du champ opératoire (1) sont réalisées de sorte que l'enroulement dudit champ opératoire autour du membre ou du corps du patient entraînant l'enroulement simultané de ladite couche externe (3) et de ladite couche interne (1),
- l'organe d'aspiration (8) est un système de récupération du sang autologue portatif ou mobile.

2. Ensemble selon la revendication 1, **caractérisé en ce que** la couche interne (4) est constituée d'une gaze neutre, de sorte à minimiser les interactions avec les globules rouges.

3. Ensemble selon l'une des revendications 1 ou 2, **caractérisé en ce que** la zone aspirante (6a) est composée d'un lacis de cathéters multi-perforés.

4. Ensemble selon l'une des revendications 1 ou 2, **caractérisé en ce que** la zone aspirante (6b) est composée de mousse multi-perforée.

5. Ensemble selon l'une des revendications 1 ou 2, **caractérisé en ce que** la zone aspirante (6c) est composée d'un réseau de treillis de cathéters.

6. Ensemble selon l'une des revendications 1 à 5, **caractérisé en ce que** le champ opératoire (1) est composé d'une partie mâle (1a) et d'une partie femelle (1b) séparables.

7. Ensemble selon l'une des revendications 1 à 6, **caractérisé en ce qu'**une fenêtre d'incision (2) totale ou partielle est emménagée dans le champ opératoire (1), de manière à avoir un accès à la plaie et/ou incision.

## Patentansprüche

1. Anordnung, aufweisend:
- eine Saugvorrichtung, die geeignet ist, auf eine Inzision und/oder eine Wunde gelegt werden kann, die sich auf einem Glied oder dem Körper eines Patienten (P) befindet, um die Inzision oder die Wunde zu reinigen und/oder zu trocknen, wobei die Vorrichtung ein Operationsfeld (1) aufweist, das Folgendes umfasst:
∘ eine Außenschicht (3), die eine Oberseite (3a) und eine Unterseite (3b) aufweist,
∘ eine Innenschicht (4), die eine Oberseite (4a) und eine Unterseite (4b) aufweist, wobei die Unterseite (4b) so angeordnet ist, dass sie mit der Wunde und/oder der Inzision in Kontakt tritt,
- eine Saugeinrichtung (8),
- einen Saugbereich (6a, 6b, 6c), der Hohlräume (61a, 61b, 61c) aufweist, wobei der Saugbereich (6a, 6b, 6c) zwischen der Unterseite (3b) der Außenschicht (3) und der Oberseite (4b) der Innenschicht (4) angeordnet ist, der Saugbereich (6a, 6b, 6c) einen dichten Rand (9) aufweist,
- eine Ablassleitung (7), die an den Saugbereich (6a, 6b, 6c) angeschlossen ist, wobei die Leitung (7) mit einem Endstück endet, das geeignet ist, mit der Saugeinrichtung (8) verbunden zu sein, um im Saugbereich (6a, 6b, 6c) einen Saugunterdruck zu erzeugen,
**dadurch gekennzeichnet, dass**:
- die Oberseite (3a) der Außenschicht (3) des Operationsfelds (1) undurchlässig ist,
- das Operationsfeld (1) geeignet ist, um das Glied oder den Körper des Patienten gewickelt zu sein,
- die Außenschicht (3) und die Innenschicht (4) des Operationsfelds (1) so ausgebildet sind, dass die Wicklung des Operationsfelds um das Glied oder den Körper des Patienten die gleichzeitige Wicklung der Außenschicht (3) und der Innenschicht (1) mit sich bringt,
- die Saugeinrichtung (8) ein tragbares oder mobiles Eigenblut-Auffangsystem ist.

2. Anordnung nach Anspruch 1, **dadurch gekennzeichnet, dass** die Innenschicht (4) von einer neutralen Gaze gebildet ist, um die Wechselwirkungen mit den roten Blutkörperchen zu minimieren.

3. Anordnung nach einem der Ansprüche 1 oder 2, **dadurch gekennzeichnet, dass** der Saugbereich (6a) von einem Lacis aus mehrfach perforierten Kathetern gebildet ist.

4. Anordnung nach einem der Ansprüche 1 oder 2, **dadurch gekennzeichnet, dass** der Saugbereich (6b) von mehrfach perforiertem Schaum gebildet ist.

5. Anordnung nach einem der Ansprüche 1 oder 2, **dadurch gekennzeichnet, dass** der Saugbereich (6c) von einem Netz von Katheter-Geflechten gebildet ist.

6. Anordnung nach einem der Ansprüche 1 bis 5, **dadurch gekennzeichnet, dass** das Operationsfeld (1) von einem männlichen Abschnitt (1a) und einem weiblichen Abschnitt (1b) gebildet ist, die trennbar sind.

7. Anordnung nach einem der Ansprüche 1 bis 6, **dadurch gekennzeichnet, dass** ein teilweises oder vollständiges Inzisionsfenster (2) im Operationsfeld (1) eingebracht ist, um einen Zugang zu der Wunde und/oder Inzision zu ermöglichen.

## Claims

1. Assembly having:
- a suctioning device suitable for being placed on an incision and/or a wound situated on a limb or on the body of a patient (P) in order to clean and/or dry said incision or said wound, said device having a surgical drape (1) comprising:
• an outer layer (3) having an upper face (3a) and a lower face (3b),
• an inner layer (4) having an upper face (4a) and a lower face (4b), which lower face (4b) is arranged in such a way as to come into contact with the wound and/or the incision,
- a suction member (8),
- a suctioning zone (6a, 6b, 6c) having cavities (61a, 61b, 61c), which suctioning zone (6a, 6b, 6c) is arranged between the lower face (3b) of the outer layer (3) and the upper face (4b) of the inner layer (4), which suctioning zone (6a, 6b, 6c) has a sealed periphery (9),
- a discharge tube (7) connected to the suctioning zone (6a, 6b, 6c), which tube (7) ends with a nozzle suitable for being connected to the suction member (8) in order to create a suction underpressure in the suctioning zone (6a, 6b, 6c),
**characterized in that**:
- the upper face (3a) of the outer layer (3) of the surgical drape (1) is impermeable,
- the surgical drape (1) is suitable for being wound around the limb or the body of the patient,
- the outer layer (3) and the inner layer (4) of the surgical drape (1) are produced such that the winding of said surgical drape around the limb or the body of the patient leads to the simultaneous winding of said outer layer (3) and of said inner layer (1),
- the suction member (8) is a portable or mobile system for recovery of autologous blood.

2. Assembly according to Claim 1, **characterized in that** the inner layer (4) is formed of a neutral gauze, so as to minimize the interactions with red blood cells.

3. Assembly according to either of Claims 1 and 2, **characterized in that** the suctioning zone (6a) is composed of an array of multi-perforated catheters.

4. Assembly according to either of Claims 1 and 2, **characterized in that** the suctioning zone (6b) is composed of multi-perforated foam.

5. Assembly according to either of Claims 1 and 2, **characterized in that** the suctioning zone (6c) is composed of a network of catheter lattices.

6. Assembly according to one of Claims 1 to 5, **characterized in that** the surgical drape (1) is composed of a male part (1a) and a female part (1b) that are separable.

7. Assembly according to one of Claims 1 to 6, **characterized in that** a total or partial incision window (2) is formed in the surgical drape (1), so as to have access to the wound and/or incision.
